# EUROPÄISCHE PATENTSCHRIFT

(11) **EP 0 969 772 B1**
(45) Veröffentlichungstag und Bekanntmachung des Hinweises auf die Patenterteilung: **30.01.2002**
(21) Anmeldenummer: 98912389.8
(22) Anmeldetag: 25.02.1998
(51) Int. Cl.: A61B 17/32

(54) **MEDIZINISCHER DISSEKTIONSSPATEL MIT SPREIZBAREN SPATELMAULTEILEN**
SPATULA DISSECTOR CAPABLE OF EXPANDABLE SPREADING
SPATULE A DISSEQUER POURVUE DE MORS ECARTABLES

(30) Priorität: 25.02.1997 DE 19707374
(43) Veröffentlichungstag der Anmeldung: 12.01.2000
(73) Patentinhaber: Karl Storz GmbH & Co. KG, 78532 Tuttlingen (DE)
(72) Erfinder: RASPALDO, Hervé, F-06400 Cannes (FR); IRION, Klaus, M., D-78576 Liptingen (DE); DITTRICH, Horst, D-78194 Immendingen (DE)
(74) Vertreter: Heuckeroth, Volker
(86) Internationale Anmeldenummer: EP9801064
(87) Internationale Veröffentlichungsnummer: WO9837817

(56) Entgegenhaltungen:
- EP-A- 0 507 622
- EP-A- 0 516 494
- EP-A- 0 702 937
- WO-A-91/13536
- WO-A-94/10920
- US-A- 5 293 863
- US-A- 5 354 313
- US-A- 5 373 840
- US-A- 5 509 923

## Beschreibung

Die vorliegende Erfindung betrifft einen medizinischen Dissektionsspatel mit einem Schaft und einem distalen flächigen Spatelende.

Ein derartiger Dissektionsspatel ist aus dem Katalog der Karl Storz GmbH & Co. "Storz - Die Welt der Endoskopie", 2. Ausgabe, Kapitel 7, 1/94, Seite NP 4 A, Nr. 28176 RE, bekannt.

Dissektionsspatel werden allgemein in der Chirurgie zum Entfernen von Gewebe durch Spaltung, Zerschneidung oder Ablösung des Gewebes von anderen Gewebeschichten verwendet. Mit dem distalen flächigen Spatelende werden dabei Gewebeschichten voneinander getrennt, Gewebeteile abgeschabt oder ähnliches. Ein solcher Spatel kann auch dazu eingesetzt werden, ein Gewebe oder Organ zu verlagern, ohne es dabei zu verletzen.

Solche Dissektionsspatel werden beispielsweise in der Schönheitschirurgie zum Ablösen von Fettgewebe unter der Haut z.B. an den Wangen, im Bereich der Augen oder im Bereich der Nasenlippenfalte eingesetzt.

Der in dem Katalog der Firma Storz GmbH & Co. unter der Nummer 28176 RE beschriebene Dissektionsspatel ist ein gerader Dissektionsspatel, dessen Schaft in ein distales, abgeflachtes, stumpfes Spatelende übergeht.

Zum Entfernen eines Gewebebereichs muß das Gewebe durch Spalten oder Abschaben von dem umliegenden Gewebe abgetrennt werden. Dies geschieht durch ein Einschieben des Spatelendes zwischen die Gewebeschichten. Dabei wird der Spatel seitlich hin- und herbewegt, und das abzutrennende Gewebe wird durch Anheben des Spatels nach und nach abgelöst. Erst dann kann ein Abschaben oder Zerschneiden des Gewebes erfolgen.

Ein Abheben und Abschaben mit dem im Stand der Technik beschriebenen geraden Dissektionsspatel ist problematisch, da das umliegende Gewebe möglichst wenig verletzt werden soll und der Spielraum für ein Hin- und Herbewegen des Spatels somit begrenzt ist. Aufgrund der starren Anordnung des Spatelendes und des Schaftes muß jedoch der gesamte Spatel bewegt werden, um das Abheben oder Abschaben zu bewerkstelligen.

Aus der US 5,293,863 ist ein Retraktor bekannt, der dem Halten und Führen von Organen bei endoskopischen Eingriffen im Körperinneren dient, um diese Organe aus dem Operationsgebiet wegzuhalten, damit diese die Operation nicht stören, oder um die freie Sicht in das hinter den Organen liegende Operationsgebiet zu schaffen. Dieser Retraktor kann auch als Dissektor verwendet werden.

Dieser bekannte Retraktor weist an seinem distalen Ende zwei spreizbare Maulteile auf, wobei beide Maulteile spreizbar sind.

Ferner ist aus der US 5,373,840 ein Endoskop zum Entfernen von Blutgefäßen bekannt, das ein Dissektionswerkzeug und ein Greifwerkzeug aufweist, das durch das Lumen des Endoskops eingeführt und dazu verwendet wird, das Blutgefäß von umgebendem Bindegewebe zu befreien. Dieses Dissektionswerkzeug weist einen Schaft mit einem Ring am distalen Ende auf, wobei der Ring zum Abstreifen des Bindegewebes vom Blutgefäß dient. Der Ring ist einstückig mit dem Schaft des Dissektionswerkzeugs verbunden. Das zuvor erwähnte Greifwerkzeug weist zwei spreizbare Maulteile auf, wobei beide Maulteile gelenkig mit einem entsprechenden Schaft des Greifwerkzeugs verbunden sind.

Vor diesem Hintergrund ist es Aufgabe der vorliegenden Erfindung, einen Dissektionsspatel bereitzustellen, mit dem Gewebe oder Gewebeteile effizient und unter größtmöglicher Vermeidung von Verletzungen abgelöst, gespalten oder getrennt werden können und mit dem eine zielgerichtete Manipulation mit einfachen Mitteln gewährleistet wird.

Diese Aufgabe wird durch einen medizinischen Dissektionsspatel der eingangs genannten Art dadurch gelöst, daß dessen Spatelende zwei Spatelmaulteile aufweist, daß die Spatelmaulteile, von der Spatelfläche weggerichtet, spreizbar sind, und daß das eine Spatelmaulteil starr mit dem Schaft verbunden und das zweite Spatelmaulteil gelenkig mit dem Schaft verbunden ist.

Durch das Vorhandensein von zwei spreizbaren Spatelmaulteilen kann das Zielgewebe nun effizient gespalten werden, indem das Spatelende mit den beiden Spatelmaulteilen im nicht gespreizten Zustand zunächst zu der beabsichtigten Stelle hingeführt wird. In dem geschlossenen bzw. nicht gespreizten Zustand arbeitet der Dissektionsspatel wie der eingangs genannte Dissektionsspatel mit nur einem Spatelteil. Durch einfaches Spreizen der Spatelmaulteile von der Spatelfläche weggerichtet kann dann das abzutrennende Gewebe von dem umgebenden Gewebe abgehoben werden. Das Ausmaß des Spreizens der beiden Spatelmaulteile richtet sich dabei nach Größe und Umfang des Gewebes, so daß der erfindungsgemäße Dissektionsspatel vielseitig einsetzbar ist. Soll der Dissektionsspatel noch weiter in das Gewebe eindringen, können die Spatelmaulteile wieder geschlossen werden und durch Hin- und Herbewegen weitere Bereiche getrennt und durch erneutes Spreizen der Spatelmaulteile voneinander abgehoben und abgelöst werden.

Durch das Vorsehen von zwei spreizbaren und somit auch schließbaren Spatelmaulteilen ist außerdem ein Ergreifen von Gewebe, oder beispielsweise auch von Adern, möglich. Es können also Körperteile, wie Adern oder Sehnen, verschoben werden, um an dahinterliegendes abzutrennendes Gewebe zu gelangen.

Sollen zwei Gewebeschichten über einen größeren Bereich hinweg voneinander getrennt werden, so kann dies mit dem erfindungsgemäßen Spatel durch Einbringen zwischen die beiden Schichten und wiederholtes Spreizen und Hin- und Herbewegen der geschlossenen Spatelmaulteile im jeweils notwendigen Ausmaß erfolgen.

Mit dem erfindungsgemäßen Dissektionsspatel werden diese Vorteile ohne aufwendige technische Vorrichtungen allein durch das Vorsehen von zwei spreizbaren Spatelmaulteilen am distalen Spatelende erreicht.

Die erfindungsgemäße Ausgestaltung mit einem unbeweglichen und einem gelenkig mit dem Schaft verbundenen Spatelmaulteil erlaubt ein besonders sicheres Arbeiten mit dem erfindungsgemäßen Dissektionsspatel, da das Spreizen mit nur einem Maulteil und daher nur in einer Richtung erfolgt. Sind zwei Gewebe beispielsweise durch eine weiche Haut oder Membran voneinander getrennt, wie dies beispielsweise zwischen Binde- und Fettgewebe oder zwischen Knochen- und Bindegewebe der Fall ist, so kann der Dissektionsspatel mit dem unbeweglichen Spatelmaulteil an die Haut angelegt werden, die nicht beeinträchtigt werden soll. Durch Spreizen des einen beweglichen Spatelmaulteils wird das von diesem abzutrennende Gewebe effizient abgelöst.

Dies erleichtert dem Operateur die Handhabung weiter deshalb, da bei geschlossenen Spatelmaulteilen der Dissektionsspatel wie ein starrer, nicht spreizbarer Spatel arbeitet und das starre Spatelmaulteil als definiertes Anlege- und Leitelement dient, von dem, wenn notwendig, das andere Spatelmaulteil weggespreizt werden kann. Dabei bleibt das starre Spatelmaulteil an einer bestimmten Stelle.

In einer bevorzugten Ausführung des erfindungsgemäßen Dissektionsspatels verbreitern sich die beiden Spatelmaulteile zu ihrem distalen Ende hin.

Hierbei ist von Vorteil, daß mit einem relativ breiten Stirnbereich größere Bereiche an Gewebe ablösbar sind. Bei einem Halten von Geweben oder Gewebeteilen, beispielsweise von Muskeln, Sehnen oder Adern ist vorteilhaft, daß diese Gewebe mit distal verbreiterten Spatelmaulteilen sicherer und atraumatischer als mit schmalen Spatelmaulteilen gehalten werden können, da sich der Haltedruck auf eine größere Haltefläche verteilt.

In einer weiteren bevorzugten Ausgestaltung liegen die beiden Spatelmaulteile, im nicht gespreizten Zustand, deckungsgleich aufeinander.

Bei dieser Ausgestaltung ist vorteilhaft, daß der Dissektionsspatel im nicht gespreizten Zustand wie ein herkömmlicher Spatel einsetzbar ist, der beim Einführen in Gewebe wenig Raum benötigt, und der dennoch am Zielort alle Vorteile entwickelt, die durch das Spreizen der Spatelmaulteile gegeben sind.

In einer weiteren bevorzugten Ausführung sind die beiden Spatelmaulteile aus der Schaftachse gekrümmt. Insbesondere sind diese über deren gesamte Länge aus der Schaftachse heraus gekrümmt.

Hierbei ist von Vorteil, daß die oft nicht geradlinig angeordneten Gewebeschichten besser erreicht werden können und daß das Ablösen von Gewebe durch Abschaben besonders leicht und effizient durchführbar ist. Bei Gesichtsoperationen können bspw. die über die gesamte Länge gekrümmten Spatelmaulteile an den gekrümmten Wangenknochen angelegt werden.

In einer höchst bevorzugten Ausgestaltung ist der Übergang zwischen dem Schaft und den sich verbreiternden Spatelmaulteilen sanft geformt.

Dies ist besonders vorteilhaft, da die Gefahr von Verletzungen beim Bewegen des Spatels vermieden wird und so ein atraumatisches Präparieren möglich ist.

In einer weiteren bevorzugten Ausgestaltung weist jedes Spatelmaulteil zwei stumpfe seitliche Kanten und eine stumpfe stimseitige Kante auf.

Hierbei ist besonders vorteilhaft, daß der erfindungsgemäße Dissektionsspatel eine atraumatische, d.h. Verletzungen vermeidende Manipulation erlaubt. Die allseitig stumpfe Begrenzung der Spatelmaulteile vermeidet dabei ein ungewünschtes Abreißen oder Zerschneiden von Gewebe, das unbeeinträchtigt bleiben soll.

In einer weiteren bevorzugten Ausführung ist zumindest ein Spatelmaulteil mit einer scharfen stirnseitigen Kante versehen.

Hierbei ist von Vorteil, daß der erfindungsgemäße Dissektionsspatel auch als chirurgisches Messer oder als Skalpell eingesetzt werden kann, so daß feste Gewebebereiche durch Schneiden mit der jeweiligen stirnseitigen Kante der Spatelmaulteile erfolgen kann. So können zum Beispiel auch Adern durchtrennt werden, oder feste Bindegewebshäute ab- oder eingeschnitten werden.

In einer bevorzugten Ausgestaltung ist die Stirnseite der Spatelmaulteile annähernd rund verlaufend ausgebildet.

Diese Form erlaubt ein sanftes Vordringen in das Gewebe.

In einer weiteren bevorzugten Ausgestaltung ist die Stirnseite der Spatelmaulteile annähernd geradlinig verlaufend ausgebildet.

Diese Ausgestaltung hat den Vorteil, daß ein Abschaben von Gewebe im Bereich von flächig ausgebildeten Geweben, beispielsweise an Häuten, besonders effizient durchführbar ist.

In einer höchst bevorzugten Ausgestaltung sind die beiden Spatelmaulteile über ein Betätigungselement spreizbar.

Dies hat den Vorteil, daß bei einem operativen Eingriff in den menschlichen Körper die Steuerung der Bewegung der Spatelmaulteile von außen möglich ist. Das Betätigungselement kann beispielsweise über ein Gelenk mit dem Spatelmaulteil verbunden sein.

In einer weiteren bevorzugten Ausgestaltung ist das Betätigungselement im Inneren des Schafts angeordnet.

Hierbei ist vorteilhaft, daß das Betätigungselement raumsparend und unter Vermeidung zusätzlicher Ecken und Kanten angebracht werden kann.

In einer höchst bevorzugten Ausführung ist der Schaft mit einem Griff verbunden, der ein fest mit dem Schaft verbundenes Griffelement und ein gelenkig mit dem ersten Griffelement und gelenkig mit dem Betätigungselement verbundenes zweites Griffelement aufweist, wobei die beiden Spatelmaulteile durch Bewegung des zweiten Betätigungselements spreizbar sind.

Diese Ausführung hat den Vorteil, daß der Chirurg den erfindungsgemäßen Dissektionsspatel einerseits mit zwei Fingern einer Hand sicher festhalten kann und gleichzeitig unter Bewegung nur eines Fingers das Spreizen der beiden Spatelmaulteile auslösen kann. So wird ein unkontrolliertes Abrutschen des Dissektionsspatels verhindert, und gleichzeitig ist ein einfaches und leicht steuerbares Spreizen im jeweilig gewünschten Ausmaß möglich.

In einer weiteren Ausgestaltung der Erfindung verläuft die Richtung der Spreizung der Spatelmaulteile etwa senkrecht zur Bewegungsebene des zweiten Griffelements.

Diese Maßnahme hat den Vorteil, daß der Operateur den geschlossenen Dissektionsspatel in einer Richtung vorantreiben kann und in derselben Richtung die Griffelemente relativ zueinander bewegen kann, ein harmonischer Vorgang, der auch überlagert werden kann. Das etwa senkrecht dazu gerichtete Spreizen löst die Gewebeteile wirkungsvoll voneinander ab und erlaubt anschließend das weitere Vorantreiben der wieder geschlossenen Spatelmaulteile. Wird beispielsweise eine Gesichtsoperation durchgeführt, bei der die Spatelmaulteile längs des Wangenknochens zur Augenhöhle vorgeführt werden, kann der Operateur durch Vorschieben des Dissektionsspatels und Bewegen der Griffelemente in derselben Richtung bzw. in entgegengesetzter Richtung sich nach und nach von außen über eine Incision längs der Wange an die Augenhöhle voranarbeiten und senkrecht zu der Vorschubrichtung durch Spreizen der Spatelmaulteile nach und nach die zu entfernenden Gewebeteile ablösen.

In einer weiteren Ausführung des erfindungsgemäßen Dissektionsspatels ist der gesamte Spatel verschieblich innerhalb eines geraden Rohrschaftelements angeordnet, wobei der lichte Innendurchmesser des Rohrschaftelements in etwa der Breite der beiden Spatelmaulteile entspricht.

Das Rohrschaftelement kann beispielsweise als Trokar ausgebildet sein.

Durch diese Ausführung wird eine Verwendung des erfindungsgemäßen Dissektionsspatels auch bei der minimal invasiven Chirurgie möglich, so daß der Spatel nicht nur bei der Dissektion von oberflächlichen Geweben eingesetzt werden kann, sondern durch das Durchführen durch einen Trokar auch Zugang zu tiefer gelegenen Organen oder Geweben erhalten wird.

In einer bevorzugten Ausgestaltung dieser Ausführung sind die beiden Spatelmaulteile des Dissektionsspatels derart ausgebildet, daß sie beim Austreten aus dem Rohrschaftelement eine Krümmung einnehmen, die die äußere Mantellinie des Rohrschaftelements seitlich überragt.

In diesem Fall können die Spatelmaulteile aus einem sogenannten "Memory-Material" hergestellt, das die Eigenschaft aufweist, reproduzierbar eine bestimmte Form nach einem Deformieren wieder einzunehmen. Es kann auch ein hochelastisches Material, wie Federstahl, eingesetzt werden. So sind die Spatelmaulteile innerhalb des Rohrschaftelements annähernd ausgestreckt, um in dem möglichst engen Rohrschaftelement Platz zu finden. Dagegen sind die beiden Spatelmaulteile nach einem Herausfahren aus dem Rohrschaftelement gekrümmt.

In einer weiteren Ausgestaltung ist der Dissektionsspatel mit einem Endoskop derart verbunden, daß der Bereich zwischen den beiden Spatelmaulteilen einsehbar ist.

Hierbei ist von Vorteil, daß der Chirurg seine Manipulation mit dem Spatelende direkt bspw. über einen Monitor verfolgen kann, wodurch ein besonders zielgerichtetes Arbeiten mit dem Dissektionsspatel möglich wird. Vor allem wenn der erfindungsgemäße Dissektionsspatel durch ein Trokar in nicht oberflächlich gelegene Körperteile eingeführt wird, erleichtert ein Endoskop die Arbeit des Chirurgen erheblich.

Dabei ist von besonderem Vorteil, wenn das Endoskop relativ zum Dissektionsspatel bewegbar, insbesondere verdrehbar, ist.

In dieser Ausgestaltung können über das Endoskop bzw. der Endoskopoptik verschieden arbeitende Bereiche des Dissektionsspatels eingesehen werden und es kann z.B. dem distalen Ende des zu spreizenden Spatelmaulteils gefolgt werden.

Weitere Vorteile ergeben sich aus der nachstehenden Beschreibung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird im folgenden an Hand der Zeichnung näher erläutert. Es zeigen:
- Fig. 1: eine Seitensicht eines erfindungsgemäßen Dissektionsspatels;
- Fig. 1a: eine um 90° verdrehte Seitenansicht des flächigen Spatelendes des Dissektionsspatels von Fig. 1, und zwar in einer ersten Betriebsstellung, bei der die Spatelmaulteile geschlossen sind; und
- Fig. 1b: eine Ansicht wie in Fig. 1b, jedoch in einer zweiten Betriebsstellung, bei der die Spatelmaulteile gespreizt sind;
- Fig. 2: eine teilweise geschnittene Seitenansicht eines Teils eines erfindungsgemäßen Dissektionsspatels, und zwar den Schaft, das distale Spatelende sowie das Betätigungselement;
- Fig. 3: eine vergrößerte ausschnittsweise Darstellung des Dissektionsspatels von Fig. 1 im Bereich der gelenkigen Verbindung von spreizbarem Spatelmaulteil und Betätigungselement;
- Fig. 4: eine weitere Ausführung des distalen Spatelendes mit scharfer Stirnkante eines erfindungsgemäßen Dissektionsspatels in einer Seitenansicht;
- Fig. 5a: eine Ausgestaltung eines erfindungsgemäßen Dissektionsspatels in Seitenansicht, der in einem Rohrschaftelement aufgenommen wird, wobei eine erste Betriebsstellung gezeigt ist, bei der das Spatelende innerhalb des Rohrschaftelements liegt;
- Fig. 5b: eine zweite Betriebsstellung, bei der das distale Spatelende ausgeschoben ist; und
- Fig. 6: eine Seitenansicht einer weiteren Ausgestaltung des erfindungsgemäßen Dissektionsspatels, der hier mit einem Endoskop verbunden ist.

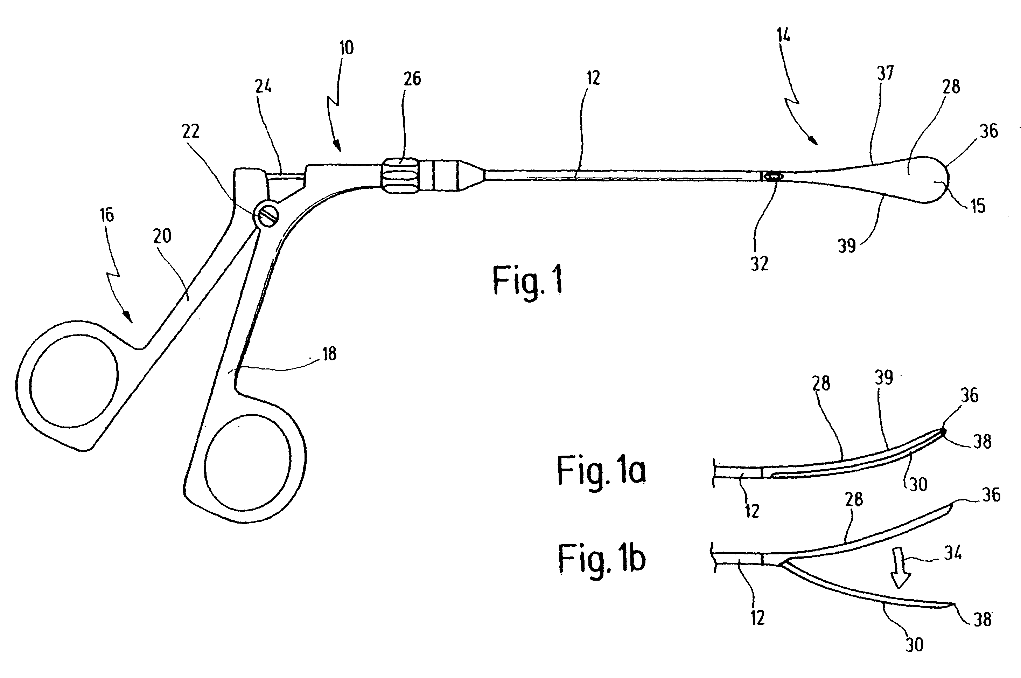

Ein in Fig. 1 gezeigter Dissektionsspatel 10 weist einen Schaft 12 mit einem flächigen, distalen Spatelende 14 auf, dessen Stirnseite 15 annähernd rund ausgebildet ist. Der Schaft 12 ist mit einem Griff 16 verbunden, der aus einem fest mit dem Schaft 12 verbundenen ersten Griffelement 18 und einem relativ zu dem ersten Griffelement 18 beweglichen zweiten Griffelement 20 besteht. Die beiden Griffelemente 18 und 20 des Griffs 16 sind über ein Scharnier 22 verbunden. Ein Betätigungselement 24 ist an dessen proximalem Ende mit dem beweglichen Griffelement 20 gelenkig verbunden.

Das Betätigungselement 24 verläuft innerhalb des Schafts 12. Der Schaft 12 samt dem distalen flächigen Spatelende 14 und dem Betätigungselement 24 können über eine Überwurfmutter 26 lösbar mit dem Griff 16 verbunden werden. Auf diese Weise ist es möglich, den Griff 16 mit verschiedenen Ausführungen des Spatelendes zu verbinden.

In Fig. 1a ist das distale Spatelende 14 in einer Seitenansicht gezeigt. In dieser Darstellung wird deutlich, daß das distale Spatelende 14 aus zwei etwa deckungsgleichen Spatelmaulteilen 28 und 30 besteht, wobei das Spatelmaulteil 28 fest mit dem Schaft 12 verbunden ist, wohingegen das Spatelmaulteil 30 über ein Gelenk 32 relativ zu dem Spatelmaulteil 28 und dem Schaft 12 verschwenkbar ist.

In Fig. 1a ist das Spatelende in einer geschlossenen Betriebsstellung abgebildet, in der die Spatelmaulteile 28 und 30 deckungsgleich aufeinanderliegen.

Die beiden aufeinanderliegenden Spatelmaulteile 28 und 30 sind aus der Schaftachse gekrümmt, wobei der Übergang zwischen Schaft 12 und Maulteilen 28 und 30 sanft verläuft.

In Fig. 1b ist eine weitere Betriebsstellung des Spatelendes 14 abgebildet, in der das Spatelmaulteil 30 in Richtung des Pfeils 34 von dem Spatelmaulteil 28 weggespreizt ist.

Die beiden Spatelmaulteile 28 und 30 weisen jeweils eine stirnseitige Kante 36 und 38 auf, die bei der in Fig. 1 abgebildeten Ausführung des Spatelendes 14 stumpf sind. Auch die jeweiligen Seitenkanten 37 und 39 sind stumpf.

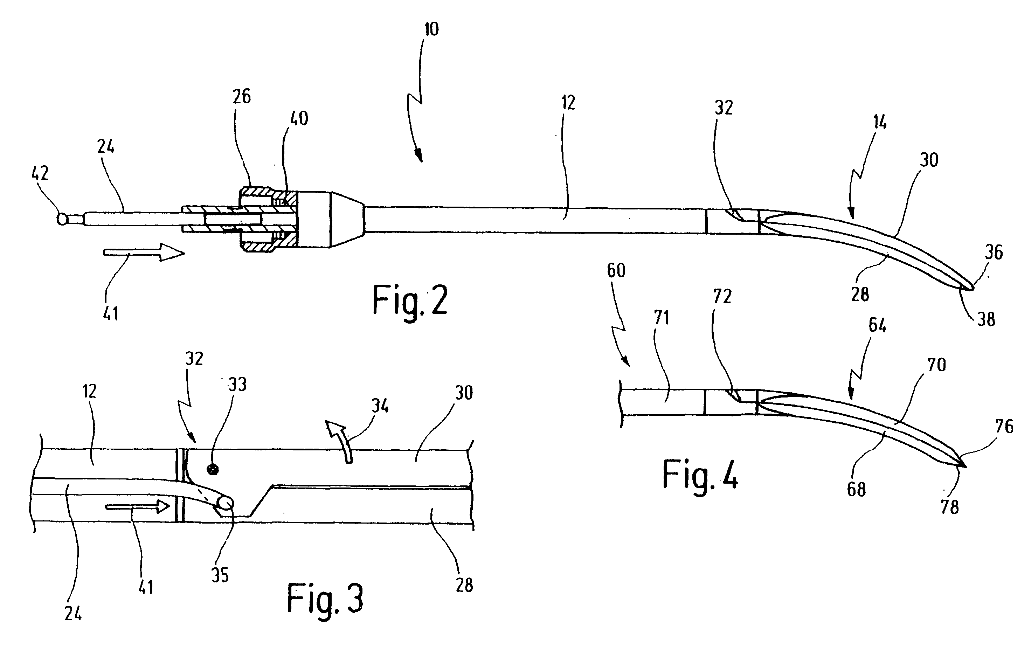

Fig. 2 zeigt den vom Griff 16 abgeschraubten Schaft 12 mit dem distalen Spatelende 14 und dem Betätigungselement 24 des in Fig. 1 gezeigten Dissektionsspatels 10 in einer teilweise angeschnittenen Seitenansicht.

Der Schaft 12 kann über die mit einem Innengewinde 40 versehene Überwurfmutter 26 an dem Griff 18 (Fig. 1) verschraubt werden.

Außerdem wird deutlich, daß das Betätigungselement 24 im Inneren des Schafts 12 verläuft. Dabei ist das Betätigungselement 24 verschieblich innerhalb des Schafts 12 angeordnet.

Wie aus Fig. 3 zu entnehmen ist, sind die beiden Spatelmaulteile 28 und 30 gelenkig über einen Achszapfen 33 miteinander verbunden, der die Schwenkachse des verschwenkbaren bzw. spreizbaren Spatelmaulteiles 30 darstellt.

Im Abstand zum Achszapfen 33 ist das Spatelmaulteil 30 über ein Auge 35 mit dem Betätigungselement 24 verbunden. Ein lineares Verschieben des Betätigungselementes 24 in Richtung eines Pfeiles 41 bewirkt ein Verschwenken (Spreizen) des Spatelmaulteiles 30, wie durch den Pfeil 34 angedeutet. Die umgekehrte Bewegung des Betätigungselementes 24 bewirkt ein Schließen der Spatelmaulteile 28 und 30. Proximal weist das Betätigungselement 24 ein kugelförmiges Ende 42 auf, das mit dem beweglichen Griffelement 20 des Griffs 16 (siehe Fig. 1) lösbar verbunden werden kann, so daß das Verschwenken des Griffelements 20 relativ zu dem fest mit dem Schaft 12 verbundenen Griffelement 18 das Verschieben des Betätigungselements 24 in distaler Richtung bewerkstelligt.

In Fig. 4 ist eine weitere Ausführung eines distalen Spatelendes gezeigt. Das Spatelende 64 weist zwei Spatelmaulteile 68 und 70 auf, die in der in Fig. 4 gezeigten Betriebsstellung deckungsgleich aufeinanderliegen (nicht gespreizter Zustand). Das Spatelmaulteil 68 ist fest mit einem Schaft 71 verbunden, wohingegen das Spatelmaulteil 70 über ein Gelenk 72 beweglich angeordnet ist. In der in Fig. 4 gezeigten Ausführung der Spatelmaulteile 70 und 68 weisen diese stirnseitige Kanten 76 und 78 auf, die scharf sind. Mit einem derartigen Spatelende ist somit ein Durchtrennen auch von festem Gewebe, wie bspw. von Adern oder Häuten, mit dem erfindungsgemäßen Dissektionsspatel möglich.

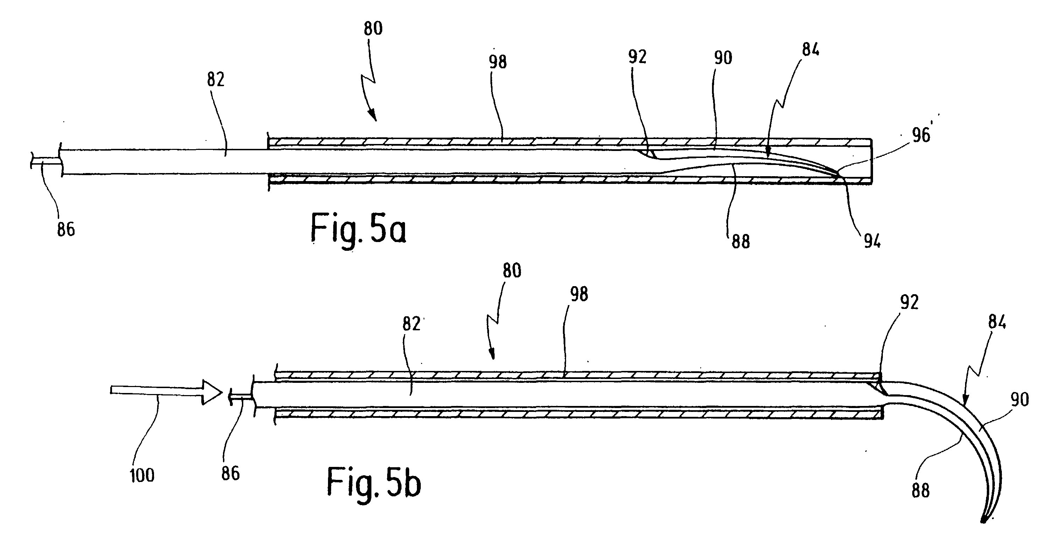

In Fig. 5a ist eine weitere Ausgestaltung eines erfindungsgemäßen Dissektionsspatels 80 gezeigt. Ein Dissektionsspatel 80, der nur teilweise gezeigt ist, weist einen Schaft 82 und ein distales Spatelende 84 auf. Das distale Spatelende 84 ist dabei aus einem fest mit dem Schaft verbundenen Spatelmaulteil 88 und einem beweglichen Spatelmaulteil 90 aufgebaut, wobei das Spatelmaulteil 90 über ein Gelenk 92 mit dem Schaft 82 und dem Spatelmaulteil 88 verbunden ist. Die Bewegung erfolgt, wie zuvor beschrieben, über ein stangenförmiges Betätigungselement 86. Die stirnseitigen Kanten 94 und 96 der Spatelmaulteile 88 und 90 sind stumpf ausgebildet. Der Schaft 82 und das Spatelende 84 des Dissektionsspatels 80 sind teilweise innerhalb eines Rohrschaftelements 98 gelegen. Das Rohrschaftelement 98 kann bspw. Teil eines Trokars sein. Der lichte Innendurchmesser des Rohrschaftelements 98 ist nicht wesentlich größer als der Durchmesser des Schafts 82 des Dissektionsspatels 80, und der Schaft 82 ist verschieblich innerhalb des Rohrschaftelements 98 angeordnet.

In Fig. 5b ist eine zweite Betriebsstellung des Dissektionsspatels 80 gezeigt, indem der Schaft 82 durch das Rohrschaftelement 98 in Richtung eines Pfeiles 100 so verschoben wurde, bis das distale Spatelende 84 aus dem Rohrschaftelement 98 herausragt. Das Spatelende 84 weist in dieser Betriebsstellung eine Krümmung auf, die die Mantellinie des Rohrschaftelements 98 seitlich weit überragt. Die Krümmung des Spatelendes 84 kommt aufgrund seiner Materialeigenschaften zustande, denn das Spatelende 84 besteht in dieser Ausführung aus einer sogenannten "Memory-Legierung", z.B. einer NiTi-Legierung, die nach dem Ausfahren wieder eine bestimmte Form einnimmt, selbst aber wieder relativ starr ist.

Das Spatelende 84 des Dissektionsspatels 80 kann durch Zurückziehen in das Rohrschaftelement 98 entgegen der Richtung des Pfeils 100 wieder verschoben werden, wobei das Spatelende 84 wieder eine annähernd ausgestreckte Form annimmt. So wird erneut die in Fig. 4a gezeigte Betriebsstellung eingenommen, und ein vollständiges Herausziehen des Dissektionsspatels 80 aus dem Rohrschaftelement 98, das bspw. im Körper des Patienten angeordnet ist, kann schonend und ohne Behinderung durch die Krümmung der Spatelmaulteile 88 und 90 bewerkstelligt werden.

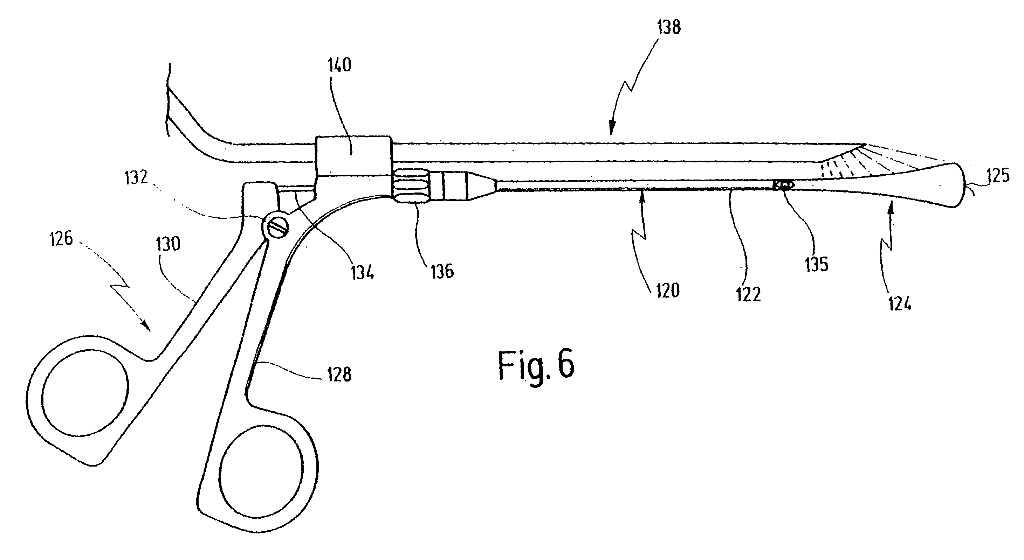

In Fig. 6 ist eine weitere Ausgestaltung des erfindungsgemäßen Dissektionsspatels gezeigt. Ein Dissektionsspatel 120 weist dabei einen Schaft 122 und ein distales Spatelende 124 auf. Die Stirnseite 125 des Spatelendes 124 ist in dieser Ausführung geradlinig ausgebildet. Der Dissektionsspatel 120 weist außerdem einen Griff 126 auf, der aus zwei Griffelementen 128 und 130 ausgebildet ist, wobei das Griffelement 128 fest mit dem Schaft 122 verbunden ist, das Griffelement 130 dagegen über ein Gelenk 132 relativ zu dem Griffelement 128 und dem Schaft 122 beweglich ist. Außerdem weist der Dissektionsspatel 120 ein Betätigungselement 134 auf, das gelenkig mit dem Griffelement 130 verbunden ist und durch Bewegen des Griffelements 130 innerhalb des Schafts 122 verschoben werden kann, wodurch das Gelenk 135 betätigt wird. Ein Verschieben des Betätigungsteils 134 bewirkt wiederum ein Spreizen der Spatelmaulteile des Spatelendes 124.

Ein Endoskop 138 ist über eine Befestigung 140 mit dem Dissektionsspatel 120 verbunden. Das Endoskop 138 ist nicht vollständig gezeigt. Durch das Endoskop 138 ist das Spatelende 124 einsehbar, und zwar auch dann, wenn die beiden Spatelmaulteile des Spatelendes 124 gespreizt sind. So ist eine dauernde Überwachung der Arbeit mit dem distalen Spatelende innerhalb des Körpers eines Patienten möglich. Das Endoskop 138 ist bewegbar, so daß bspw. dem distalen Ende des spreizbaren Spatelmaulteils beim Spreizen gefolgt werden kann.

Die in Fig. 5a und 5b gezeigte Ausführung kann ebenfalls mit einer Endoskopoptik versehen sein, die am oder im Rohrschaftelement vorgesehen ist.

## Patentansprüche

1. Medizinischer Dissektionsspatel (10; 60; 80; 120) mit einem Schaft (12; 71; 82; 122) und einem distalen flächigen Spatelende (14; 64; 84; 124), **dadurch gekennzeichnet, daß** das Spatelende (14; 64; 84; 124) zwei Spatelmaulteile (28, 30; 68, 70; 88, 90) aufweist, daß die Spatelmaulteile (28, 30; 68, 70; 88, 90) von der Spatelfläche weggerichtet spreizbar sind, und daß das eine Spatelmaulteil (28; 68; 88) starr mit dem Schaft (12; 71; 82; 122) und das zweite Spatelmaulteil (30; 70; 90) gelenkig mit dem Schaft (12; 71; 82; 122) verbunden ist.

2. Dissektionsspatel nach Anspruch 1, **dadurch gekennzeichnet, daß** sich die beiden Spatelmaulteile (28, 30; 68, 70; 88, 90) in Richtung deren distalen Endes (15; 125) hin verbreitern.

3. Dissektionsspatel nach Anspruch 1 oder 2, **dadurch gekennzeichnet, daß** die beiden Spatelmaulteile (28, 30; 68, 70; 88, 90) im nicht gespreizten Zustand deckungsgleich aufeinanderliegen.

4. Dissektionsspatel nach einem der Ansprüche 1 bis 3, **dadurch gekennzeichnet, daß** die beiden Spatelmaulteile (28, 30; 68, 70; 88, 90) aus der Schaftachse heraus gekrümmt sind.

5. Dissektionsspatel nach Anspruch 4, **dadurch gekennzeichnet, daß** die beiden Spatelmaulteile (28, 30; 68, 70; 88, 90) über deren gesamte Länge aus der Schaftachse heraus gekrümmt sind.

6. Dissektionsspatel nach einem der Ansprüche 2 bis 5, **dadurch gekennzeichnet, daß** der Übergang zwischen dem Schaft (12; 71; 82; 122) und den sich verbreiternden Spatelmaulteilen (28, 30; 68, 70; 88, 90) sanft geformt ist.

7. Dissektionsspatel nach einem der Ansprüche 1 bis 6, **dadurch gekennzeichnet, daß** jedes Spatelmaulteil (28, 30; 68, 70; 88, 90) zwei stumpfe seitliche Kanten (37, 39) aufweist.

8. Dissektionsspatel nach einem der Ansprüche 1 bis 7, dädurch **gekennzeichnet**, daß jedes Spatelmauiteil (28, 30; 68, 70; 88, 90) eine stumpfe stirnseitige Kante (36, 38; 94, 96) aufweist.

9. Dissektionsspatel nach einem der Ansprüche 1 bis 7, **dadurch gekennzeichnet, daß** zumindest ein Spatelmaulteil (68, 70) eine scharfe stirnseitige Kante (76; 78) aufweist.

10. Dissektionsspatel nach einem der Ansprüche 7 bis 9, **dadurch gekennzeichnet, daß** die Stirnseite (15) der Spatelmaulteile (28, 30) annähernd rund verlaufend ausgebildet ist.

11. Dissektionsspatel nach einem der Ansprüche 7 bis 9, **dadurch gekennzeichnet, daß** die Stirnseite (125) der Spatelmaulteile annähernd geradlinig verlaufend ausgebildet ist.

12. Dissektionsspatel nach einem der Ansprüche 1 bis 11, **dadurch gekennzeichnet, daß** die beiden Spatelmaulteile (28, 30; 68, 70; 88, 90) über ein Betätigungselement (24; 134) spreizbar sind.

13. Dissektionsspatel nach Anspruch 12, **dadurch gekennzeichnet, daß** das Betätigungselement (24; 134) im Inneren des Schafts (12; 71; 82; 122) angeordnet ist.

14. Dissektionsspatel nach einem der Ansprüche 11 bis 13, **dadurch gekennzeichnet, daß** der Schaft mit einem Griff (16; 126) verbunden ist, der ein fest mit dem Schaft (12; 71; 82; 122) verbundenes Griffelement (18; 128) und ein gelenkig mit dem ersten Griffelement (18; 128) und gelenkig mit dem Betätigungselement (24; 134) verbundenes zweites Griffelement (20; 130) aufweist, wobei die beiden Spatelmaulteile (28, 30; 68, 70; 88, 90) durch Bewegung des zweiten Griffelements (20; 130) spreizbar sind.

15. Dissektionsspatel nach Anspruch 14, **dadurch gekennzeichnet, daß** die Richtung (Pfeil 34) der Spreizung der Spatelmaulteile etwa senkrecht zur Bewegungsebene des zweiten Griffelements (20; 130) ist.

16. Dissektionsspatel nach einem der Ansprüche 1 bis 15, **dadurch gekennzeichnet, daß** er verschieblich innerhalb eines geraden Rohrschaftelements (98) angeordnet ist, dessen lichter Innendurchmesser in etwa der Breite der beiden Spatelmaulteile (88, 90) entspricht.

17. Dissektionsspatel nach Anspruch 16, **dadurch gekennzeichnet, daß** die beiden Spatelmaulteile (88, 90) derart ausgebildet sind, daß sie beim Austreten aus dem Rohrschaftelement (98) eine Krümmung einnehmen, die die Mantellinie des Rohrschaftelements (98) seitlich überragt.

18. Dissektionsspatel nach einem der Ansprüche 1 bis 17, **dadurch gekennzeichnet, daß** er mit einem Endoskop (138) derart verbunden ist, daß der Bereich zwischen den beiden Spatelmaulteilen (28, 30; 68, 70; 88, 90) einsehbar ist.

19. Dissektionsspatel nach Anspruch 18, **dadurch gekennzeichnet, daß** das Endoskop (138) relativ zum Dissektionsspatel bewegbar, insbesondere drehbar, ist.

## Claims

1. A medical dissection spatula (10; 60; 80; 120), comprising a shaft (12; 71; 82; 122) and a distal flat spatula end (14; 64; 84; 124), **characterized in that** the spatula end (14; 64; 84; 124) has two spatula jaw parts (28, 30; 68, 70; 88, 90), the spatula jaw parts (28, 30; 68, 70; 88, 90) are spreadable out away from the spatula surface, and one of the two spatula jaw parts (28; 68; 88) is joined immovably to the shaft (12; 71; 82; 122), and the second spatula jaw part (30; 70; 90) is joined articulatedly to the shaft (12; 71; 82; 122).

2. The dissection spatula of Claim 1, **characterized in that** the two spatula jaw parts (28, 30; 68, 70; 88, 90) broaden toward their distal end (15; 125).

3. The dissection spatula of Claim 1 or 2, **characterized in that** the two spatula jaw parts (28, 30; 68, 70; 88, 90) rest congruently on one another in the unspread state.

4. The dissection spatula of anyone of Claims 1 through 3, **characterized in that** the two spatula jaw parts (28, 30; 68, 70; 88, 90) are curved out of the shaft axis.

5. The dissection spatula of Claim 4, **characterized in that** the two spatula jaw parts (28, 30; 68, 70; 88, 90) are curved out of the shaft axis over their entire length.

6. The dissection spatula of anyone of Claims 2 through 5, **characterized in that** the transition between the shaft (12; 71; 82; 122) and the broadening spatula jaw parts (28, 30; 68, 70; 88, 90) is smoothly shaped.

7. The dissection spatula of anyone of Claims 1 through 6, **characterized in that** each spatula jaw part (28, 30; 68, 70; 88, 90) has two blunt lateral edges (37, 39).

8. The dissection spatula of anyone of Claims 1 through 7, **characterized in that** each spatula jaw part (28, 30; 68, 70; 88, 90) has one blunt front edge (36, 38; 94, 96).

9. The dissection spatula of anyone of Claims 1 through 8, **characterized in that** at least one spatula jaw part (68, 70) is equipped with a sharp front edge (76; 78).

10. The dissection spatula of anyone of Claims 7 through 9, **characterized in that** the front face (15) of the spatula jaw parts (28, 30) is configured with an approximately round profile.

11. The dissection spatula of anyone of Claims 7 through 9, **characterized in that** the front face (125) of the spatula jaw parts is configured with an approximately straight profile.

12. The dissection spatula of anyone of Claims 1 through 11, **characterized in that** the two spatula jaw parts (28, 30; 68, 70; 88, 90) are spreadable by way of an actuation element (24; 134).

13. The dissection spatula of Claim 12, **characterized in that** the actuation element (24; 134) is arranged in the interior of the shaft (12; 71; 82; 122).

14. The dissection spatula of anyone of Claims 11 through 13, **characterized in that** the shaft is joined to a handle (16; 126) that has one grip element (18; 128) joined immovably to the shaft (12; 71; 82; 122) and a second grip element (20; 130) joined in articulated fashion to the first grip element (18; 128) and in articulated fashion to the actuation element (24; 134), the two spatula jaw parts (28, 30; 68, 70; 88, 90) being spreadable by moving the second grip element (20; 130).

15. The dissection spatula of Claim 14, **characterized in that** the spreading direction (arrow 34) of the spatula jaw parts is approximately perpendicular to the movement plane of the second grip element (20; 130).

16. The dissection spatula of anyone of Claims 1 through 15, **characterized in that** it is arranged displaceably within a straight tubular shaft element (98) whose inner diameter corresponds approximately to the width of the two spatula jaw parts (88, 90).

17. The dissection spatula of Claim 16, **characterized in that** the two spatula jaw parts (88, 90) are configured in such a way that upon emerging from the tubular shaft element (98), they assume a curvature that projects laterally beyond the outer surface line of the tubular shaft element (98).

18. The dissection spatula of anyone of Claims 1 through 17, **characterized in that** it is joined to an endoscope (138) in such a way that the region between the two spatula jaw parts (28, 30; 68, 70; 88, 90) is visible.

19. The dissection spatula of Claim 18, **characterized in that** the endoscope (138) is movable, in particular is rotatable, relative to the dissection spatula.

## Revendications

1. Spatule de dissection médicale (10 ; 60 ; 80 ; 120) comportant une tige (12 ; 71 ; 82 ; 122) et une extrémité distale de spatule (14 ; 64 ; 84 ; 124) plate, **caractérisée en ce que** l'extrémité de spatule (14 ; 64 ; 84 ; 124) comporte deux parties de mâchoire (28, 30 ; 68, 70 ; 88, 90), **en ce que** les parties de mâchoire de spatule (28, 30 ; 68, 70 ; 88, 90) peuvent s'écarter en s'éloignant de la surface de spatule, et **en ce qu'**une partie de mâchoire de spatule (28, 68 ; 88) est reliée rigidement à la tige (12 ; 71 ; 82 ; 122) et la deuxième partie de mâchoire de spatule (30 ; 70 ; 90) est reliée de manière articulée à la tige (12 ; 71 ; 82 ; 122).

2. Spatule de dissection selon la revendication 1, **caractérisée en ce que** les deux parties de mâchoire de spatule (28, 30 ; 68, 70 ; 88, 90) s'élargissent en direction de leur extrémité distale (15 ; 125).

3. Spatule de dissection selon la revendication 1 ou 2, **caractérisée en ce que** les deux parties de mâchoire de spatule (28, 30 ; 68, 70 ; 88, 90) reposent l'une sur l'autre de manière à se recouvrir à l'état non écarté.

4. Spatule de dissection selon l'une des revendications 1 à 3, **caractérisée en ce que** les deux parties de mâchoire de spatule (28, 30 ; 68, 70 ; 88, 90) sont courbées à l'extérieur de l'axe de la tige.

5. Spatule de dissection selon la revendication 4, **caractérisée en ce que** les deux parties de mâchoire de spatule (28, 30 ; 68, 70 ; 88, 90) sont courbées sur toute leur longueur à l'extérieur de l'axe de la tige.

6. Spatule de dissection selon l'une des revendications 2 à 5, **caractérisée en ce que** la transition entre la tige (12; 71 ; 82 ; 122) et les parties de mâchoire de spatule (28, 30 ; 68, 70 ; 88, 90) s'élargissant est de forme douce.

7. Spatule de dissection selon l'une des revendications 1 à 6, **caractérisée en ce que** chaque partie de mâchoire de spatule (28, 30 ; 68, 70 ; 88, 90) présente deux bords latéraux (37, 39) émoussés.

8. Spatule de dissection selon l'une des revendications 1 à 7, **caractérisée en ce que** chaque partie de mâchoire de spatule (28, 30 ; 68, 70 ; 88, 90) présente un bord frontal (36, 38 ; 94, 96) émoussé.

9. Spatule de dissection selon l'une des revendications 1 à 7, **caractérisée en ce qu'**au moins une partie de mâchoire de spatule (68, 70) présente une arête frontale (76 ; 78) vive.

10. Spatule de dissection selon l'une des revendications 7 à 9, **caractérisée en ce que** le côté frontal (15) des parties de mâchoire de spatule (28, 30) s'étend approximativement en cercle.

11. Spatule de dissection selon l'une des revendications 7 à 9, **caractérisée en ce que** le côté frontal (125) des parties de mâchoire de spatule s'étend approximativement en ligne droite.

12. Spatule de dissection selon l'une des revendications 1 à 11, **caractérisée en ce que** les deux parties de mâchoire de spatule (28, 30 ; 68, 70 ; 88, 90) peuvent être écartées au moyen d'un élément d'actionnement (24 ; 134).

13. Spatule de dissection selon la revendication 12, **caractérisée en ce que** l'élément d'actionnement (24 ; 134) est disposé à l'intérieur de la tige (12 ; 71 ; 82 ; 122).

14. Spatule de dissection selon l'une des revendications 11 à 13, **caractérisée en ce que** la tige est reliée à une poignée (16 ; 126) qui comporte un élément de prise (18 ; 128), relié fixement à la tige (12 ; 71 ; 82 ; 122) ainsi qu'un deuxième élément de prise (20 ; 130) relié de manière articulée au premier élément de prise (18; 128) et de manière articulée à l'élément d'actionnement (24 ; 134), les deux parties de mâchoire de spatule (28, 30; 68, 70 ; 88, 90) pouvant être écartées par mouvement du deuxième élément de prise (20 ; 130).

15. Spatule de dissection selon la revendication 14, **caractérisée en ce que** le sens (flèche 34) de l'écartement des parties de mâchoire de spatule est à peu près perpendiculaire au plan de mouvement du deuxième élément de prise (20 ; 130).

16. Spatule de dissection selon l'une des revendications 1 à 15, **caractérisée en ce qu'**elle est disposée de manière à pouvoir coulisser à l'intérieur d'un élément de tige tubulaire (98) droit dont le diamètre intérieur libre correspond approximativement à la largeur des deux parties de mâchoire de spatule (88, 90).

17. Spatule de dissection selon la revendication 16, **caractérisée en ce que** les deux parties de mâchoire de spatule (88, 90) sont réalisées de manière que lorsqu'elles sortent de l'élément de tige tubulaire (98) elles prennent une courbure qui dépasse latéralement de la génératrice de l'élément de tige tubulaire (98).

18. Spatule de dissection selon l'une des revendications 1 à 17, **caractérisée en ce qu'**elle est reliée à un endoscope (138) de manière que la zone entre les deux parties de mâchoire de spatule (28, 30 ; 68, 70 ; 88, 90) soit visible.

19. Spatule de dissection selon la revendication 18, **caractérisée en ce que** l'endoscope (138) peut se déplacer par rapport à la spatule de dissection, en particulier tourner.
